(19) Europäisches Patentamt
European Patent Office
Office européen des brevets

(11) Veröffentlichungsnummer : **0 035 723**
**B1**

(12) # EUROPÄISCHE PATENTSCHRIFT

(45) Veröffentlichungstag der Patentschrift : **05.10.83**

(51) Int. Cl.³ : **C 07 D307/52**, C 07 D333/20, C 07 C143/80, A 61 K 31/63

(21) Anmeldenummer : **81101462.0**

(22) Anmeldetag : **28.02.81**

(54) **2,4-Diamino-5-sulfamoylbenzolsulfosäuren und Verfahren zu ihrer Herstellung.**

(30) Priorität : **11.03.80 DE 3009229**

(43) Veröffentlichungstag der Anmeldung : **16.09.81 Patentblatt 81/37**

(45) Bekanntmachung des Hinweises auf die Patenterteilung : **05.10.83 Patentblatt 83/40**

(84) Benannte Vertragsstaaten : **AT BE CH DE FR GB IT LI NL SE**

(56) Entgegenhaltungen :
**DE A 2 232 457**
**DE A 2 406 972**
**DE A 2 453 548**
**DE A 2 718 871**

**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Sturm, Karl, Dr.**
**Berndesallee 64**
**D-6501 Heidesheim (DE)**
Erfinder : **Muschaweck, Roman, Dr.**
**Helmchenweg 39**
**D-6230 Frankfurt am Main 80 (DE)**

**EP 0 035 723 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

2,4-Diamino-5-sulfamoylbenzolsulfosäuren und Verfahren zu ihrer Herstellung

Gegenstand der Erfindung sind Verbindungen der Formel I, die der Gruppe der substituierten 2,4-Diamino-5-sulfamoylbenzolsulfosäuren zuzuordnen sind, sowie deren physiologisch verträglichen Salze

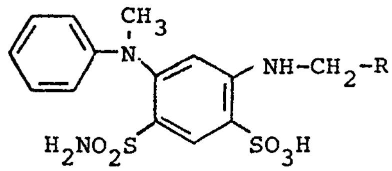

(I)

In der Formel bedeutet : R Furyl, Thienyl oder Phenyl.

Als R sind in erster Linie 2-Furyl und 2-Thienyl von Interesse.

Zur Salzbildung eignen sich alle physiologisch verträglichen Kationen, insbesondere Alkali- oder Erdalkaliionen, das Ammoniumion oder substituierte Ammoniumionen.

Gegenstand der Erfindung ist ferner ein Verfahren zur Herstellung der Verbindungen der Formel I. Das Verfahren ist dadurch gekennzeichnet, daß man Verbindungen der Formel II

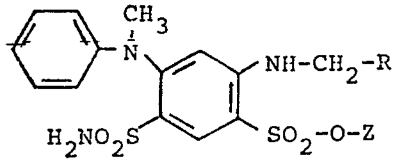

(II)

in der Z Aryl bedeutet, alkalisch verseift, und gegebenenfalls die erhaltene Verbindung in die freie Säure oder ein Salz überführt.

Als Rest Z im Ausgangsmaterial der Formel II kommt prinzipiell jeder aromatische Rest infrage ; für die technische Synthese verwendet man jedoch mit besonderem Vorteil die einfach herzustellenden Phenyl- oder Kresylester.

Die alkalische Verseifung der Ester wird vorzugsweise im wäßrigen Medium mit einer anorganischen Base, insbesondere mit überschüssiger 1- bis 5n Natronlauge oder Kalilauge, ausgeführt.

Zur Herstellung der in der Literatur noch nicht beschriebenen Ausgangsstoffe mit der allgemeinen Formel II geht man vorteilhaft aus von einem 2,4-Dichlor-sulfamoylbenzolsulfosäureester der Formel III

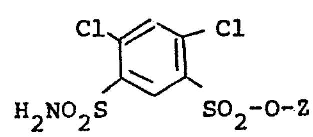

(III)

worin Z vorzugsweise einen Phenyl- oder Tolylrest bedeutet. Die Herstellung solcher Ester ist in der Deutschen Bundespatentschrift 2 718 871 beschrieben.

In erster Stufe setzt man eine Dichlorverbindung der Formel III bei einer Temperatur zwischen 120 und 140 °C mit N-Methylanilin um, wobei man vorteilhaft mit einem Überschuss der Base und ohne Zusatz eines Lösungsmittels arbeitet.

Das erhaltene N-Methylanilinoderivat der Formel IV

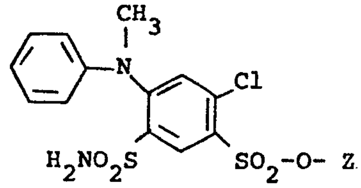

(IV)

liefert bei der nachfolgenden Umsetzung mit einem Amin der Formel $R—CH_2—NH_2$, vorteilhaft im Überschuß und bei einer Temperatur zwischen 120 und 140 °C, den gewünschten Ausgangsstoff der Formel II.

Die alkalische Verseifung der Ester der Formel II zu den Endprodukten der Formel I wird vorzugsweise mit anorganischen Basen, besonders vorteilhaft mit wäßriger Natronoder Kalilauge ausgeführt. Beispielsweise können unsubstituierte Phenylester der allgemeinen Formel II durch 2n Natron- oder Kalilauge unter Rückfluß innerhalb von 2-3 Stunden verseift werden. Ester mit höhermolekularer

Phenolkomponente erfordern unter Umständen einen Zusatz eines mit Wasser mischbaren organischen Lösungsmittels wie Dioxan, Glykolmonomethyläther oder Diglyme und/oder eine Verlängerung der Reaktionszeit. Nach beendeter Verseifung stellt man die Reaktionslösung, vorteilhaft mit verdünnter Salzsäure, auf einen pH-Wert zwischen 7 und 8 ein, worauf sich das Natrium- bzw. Kaliumsalz des Endproduktes bereits bei Raumtemperatur kristallin abscheidet.

War das eingesetzte Ausgangsmaterial der Formel II ein unsubstituierter Phenylester, bleibt das gebildete Phenol in der wäßrigen Mutterlauge und das abgenutschte, mit Wasser gewaschene Produkt ist bereits sehr rein. Ist bei der Verseifung ein in Wasser schwerer lösliches Phenol entstanden, wird das luftgetrocknete Rohprodukt durch Extraktion mit einem geeigneten organischen Lösungsmittel wie beispielsweise Diäthyläther, Diisopropyläther, Toluol oder Essigester von dem betreffenden Phenol befreit. Zur Endreinigung wird aus Wasser oder einer Alkohol-Wasser-Mischung umkristallisiert.

Für therapeutische Zwecke werden vorzugsweise die relativ gut und mit schwach alkalischer Reaktion in Wasser löslichen Alkalisalze, vorzugsweise die Natrium- oder Kaliumsalze der erfindungsgemäßen Verbindungen verwendet. Die am leichtesten wasserlöslichen Natriumsalze kommen vor allem für Injektionslösungen in Frage, während die schwerer löslichen Kaliumsalze für alle oral anwendbaren galenischen Zubereitungen besonders geeignet sind.

Für spezielle therapeutische Zwecke können auch Calcium-, Magnesium- oder Ammoniumsalze der Verbindungen geeignet sein. Diese Salze werden vorteilhaft aus einer wäßrigen Lösung des Natriumsalzes durch Zugabe eines mehrfach molekularen Überschusses Calciumchlorid, Magnesiumchlorid oder des betreffenden Aminhydrochlorids kristallin ausgefällt, oder, falls sie leichter wasserlöslich sind als das Natriumsalz, unter Verwendung eines Ionenaustauschers erhalten.

Besondere pharmakologische Bedeutung haben auch die Salze der erfindunsgemäßen Verbindungen mit basischen, kaliumretinierenden Verbindungen wie beispielsweise Amilorid oder Triamteren oder mit basichen Antihypertensiva wie beispielsweise Clonidin, Dihydralazin oder β-Blockern.

In Form der freien Säure sind die Verbindungen nicht sehr stabil. Falls sie in dieser Form gewünscht werden, kann man beispielsweise eine wäßrige Lösung des Natriumsalzes über einen sauren Ionenaustauscher filtrieren und das Filtrat gefriertrocknen.

Die erfindungsgemäßen Verbindungen sind ausgezeichnete Salidiuretika vom Furosemid-Typ mit außerordentlich hoher Potenz, die außerdem eine besonders niedrige Kaliumausscheidung und eine starke uricosurische Wirkung aufweisen. Beispielsweise zeichnet sich das gemäss Beispiel 1 erhältliche 2-Furfurylamino-4-(N-methylanilino)-5-sulfamoylbenzolsulfonsäure-natrium im Vergleich zu der aus der DE-A 27 18 871 bekannten 4-Phenoxyverbindung durch seine überraschend hohe salidiuretische Aktivität und seine deutlich stärkere uricosurische Wirkung aus.

Für die Humantherapie kommen für die orale Applikation vorzugsweise Tabletten, Dragees oder Kapseln mit 0,1 bis 50 mg Wirkstoff in Frage.

## Beispiele

### Beispiel 1

2-Furfurylamino-4-(N-methylanilino)-5-sulfamoylbenzolsulfosäure-natrium

51,4 g 2-Furfurylamino-4-(N-methylanilino)-5-sulfamoylbenzolsulfosäurephenylester (0,1 Mol) werden mit 0,4 l 2n NaOH 3 Stunden rückfließend erhitzt. Die auf Raumtemperatur abgekühlte und filtrierte Reaktionslösung wird mit 2n HCl auf pH 7 eingestellt, die kristalline Fällung nach einstündigem Stehen bei Raumtemperatur abgesaugt und mit wenig Eiswasser gewaschen. Das schwach gefärbte nutschfeuchte Produkt wird nachfolgend aus Wasser, unter Verwendung von Aktivkohle, umkristallisiert und auf dem Dampfbad getrocknet.

36 g farblose Prismen (79 % d. Th.), Zers.-P. 195 °C

Ausgangsmaterial:

383 g 2,4-Dichlor-5-sulfamoylbenzolsulfosäurephenylester (1,0 Mol) werden mit 500 ml frisch destilliertem N-Methylanilin 8 Stunden unter Stickstoff bei 130 °C gerührt. Man verdünnt die auf 50 °C abgekühlte Reaktionslösung mit 0,4 l Methanol und rührt die Mischung in 5 l 2n HCl ein. Die amorphe Fällung wird dekantierend abgetrennt, aus 90-proz. Methanol umkristallisiert und nach dem Waschen mit Äthanol auf dem Dampfbad getrocknet.

220 g 2-Chlor-4-(N-methylanilino)-5-sulfamoylbenzosulfosäurephenylester (49 % d. Th.), Schmp. 139-141 °C. 182 g dieses Esters (0,4 Mol) werden mit 0,5 l frisch destilliertem Furylfurylamin 2 Stunden bei 125 °C gerührt und die Reaktionslösung dann in 5 l 10-proz. Essigsäure eingerührt. Die kristalline Fällung wird abgesaugt, gründlich mit Wasser gewaschen und luftgetrocknet.

190 g 2-Furfurylamino-4-(N-methylanilino)-5-sulfamoylbenzolsulfosäurephenylester (94 % d. Th.), ab 80 °C Sintern. Das Produkt ist chromatographisch einheitlich und kann direkt in die Verseifung eingesetzt werden.

Beispiel 2

2-(2-Thienylmethylamino)-4-(N-methylanilino)-5-sulfamoylbenzolsulfosäure-natrium

53 g 2-(2-Thienylmethylamino)-4-(N-methylanilino)-5-sulfamoylbenzolsulfosäurephenylester (0,1 Mol) werden mit 0,5 l 2n NaOH 3 Stunden rückflußgekocht. Nach dem Abkühlen auf Raumtemperatur wird das Endprodukt mit 2n HCl bei pH 7 kristallin gefällt, auf der Nutsche mit Wasser und wenig Äthanol gewaschen und bei 100 °C getrocknet.

45 g farblose Kristalle (95 % d. Th.), Zers.-P. 215 °C.

Ausgangsmaterial :

182 g 2-Chlor-4-(N-methylanilino)-5-sulfamoylbenzolsulfosäurephenylester (0,4 Mol) werden analog Beispiel 1 mit 0,5 l 2-Thienylmethylamin umgesetzt. Bei analoger Aufarbeitung erhält man in chromatographisch reiner Form.

200 g 2-(2-Thienylmethylamino)-4-(N-methylanilino)-5-sulfamoylbenzolsulfosäure-phenylester (94 % d. Th.), ab 90 °C Sint.

Beispiel 3

2-Benzylamino-4-(N-methylanilino)-5-sulfamoylbenzolsulfosäure-natrium

52,4 g 2-Benzylamino-4-(N-methylanilino)-5-sulfamoylbenzolsulfosäurephenylester (0,1 Mol) werden analog Beispiel 2 mit 2n NaOH verseift und das Endprodukt wie dort beschrieben, isoliert.

40 g farblose Kristalle (85 % d. Th.), Zers.-P. 245 °C.

Ausgangsmaterial :

Umsetzung von 2-Chlor-4-(N-methylanilino)-5-sulfamoylbenzolsulfosäurephenylester mit Benzylamin, analog Beispiel 1.

**Ansprüche** (für die Vertragsstaaten : BE, CH, DE, FR, GB, IT, LI, NL, SE)

1. Verbindungen der Formel I

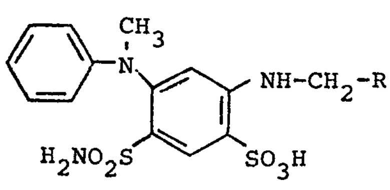

(I)

in der R Furyl, Thienyl oder Phenyl bedeutet sowie deren physiologisch verträgliche Salze.

2. Verbindungen gemäß Anspruch 1, worin R 2-Furyl oder Thienyl ist.

3. Verfahren zur Herstellung von Verbindungen gemäß Anspruch 1 oder 2, dadurch gekennzeichnet, daß man eine Verbindung der Formel II

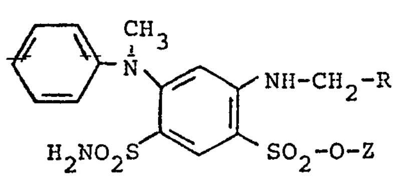

(II)

in der
R Furyl, Thienyl oder Phenyl und
Z aryl bedeutet, alkalisch verseift.

4. Heilmittel, enthaltend eine Verbindung gemäß Anspruch 1 neben üblichen Träger- und Hilfsstoffen.

5. Verbindung gemäß Anspruch 1 zur Verwendung als Heilmittel.

**Anspruch** (für den Vertragsstaat AT)

Verfahren zur Herstellung von Verbindungen der Formel I

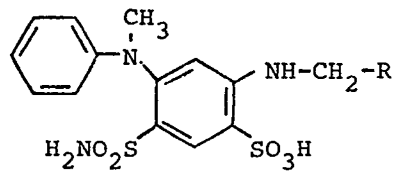

(I)

in der R Furyl, Thienyl oder Phenyl bedeutet sowie von deren physiologisch verträglichen Salzen, dadurch gekennzeichnet, daß man eine Verbindung der Formel II

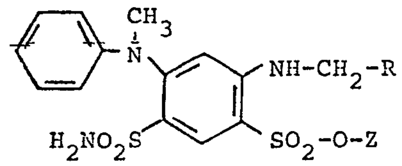

(II)

worin R die obige Bedeutung hat und Z Aryl bedeutet, alkalisch verseift.

**Claims** (for the Contracting States : BE, CH, DE, FR, GB, IT, LI, NL, SE)

1. Compounds of the formula I

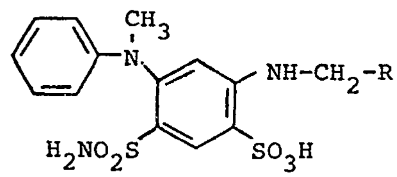

(I)

wherein R is furyl, thienyl or phenyl and physiologically acceptable salts thereof.

2. Compounds as claimed in claim 1, wherein R is 2-furyl or 2-thienyl.

3. A process for the manufacture of compounds as claimed in any of claims 1 or 2, characterized in that a compound of formula II

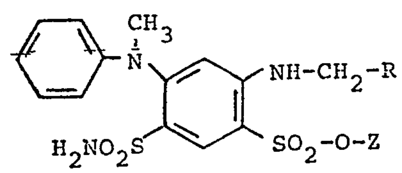

(II)

wherein

R is furyl, thienyl or phenyl and

Z is aryl, is subjected to an alkaline saponification.

4. Medicament comprising a compound according to claim 1 in admixture with pharmaceutically suitable carriers and auxiliaries.

5. Compound according to claim 1 for use as medicament.

**Claim** (for the Contracting State AT)

A process for the manufacture of compounds of the formula I

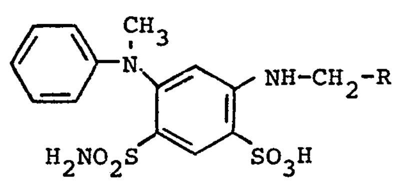

(I)

wherein R is furyl, thienyl or phenyl and physiologically acceptable salts thereof, characterized in that a compound of formula II

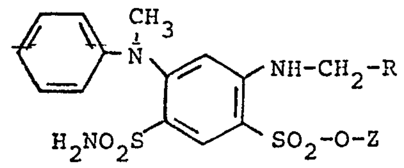 (II)

wherein R is a defined above and Z is aryl, is subjected to an alkaline saponification.

**Revendications** (pour les Etats contractants : BE, CH, DE, FR, GB, IT, LI, NL, SE)

1. Composés répondant à la formule I

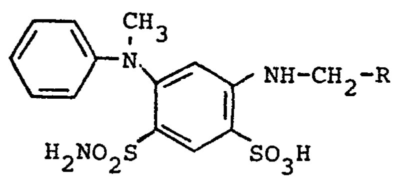 (I)

dans laquelle R représente un groupe furyle, thiényle ou phényle, ainsi que leurs sels physiologiquement acceptables.

2. Composés selon la revendication 1, dans lesquels R représente le groupe 2-furyle ou thiényle.

3. Procédé de préparation de composés selon la revendication 1 ou 2, caractérisé en ce qu'on saponifie par un alcali un composé de formule II

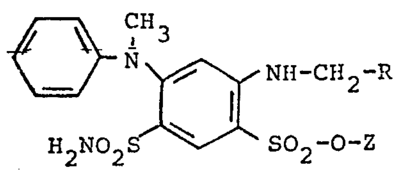 (II)

dans laquelle

R représente un groupe furyle, thiényle ou phényle et

Z représente un groupe aryle.

4. Médicament contenant un composé selon la revendication 1, en association avec des véhicules et excipients usuels.

5. Composé selon la revendication 1 pour utilisation comme médicament.

**Revendication** (pour l'Etat contractant AT)

Procédé pour la préparation de composés de formule I

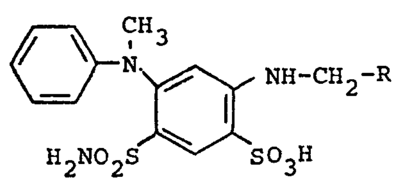 (I)

dans laquelle R représente un groupe furyle, thiényle ou phényle, ainsi que leurs sels physiologiquement acceptables, caractérisé en ce qu'on saponifie par un alcali un composé de formule II

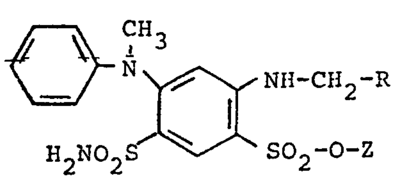 (II)

dans laquelle R a la signification ci-dessus et Z représente un groupe aryle.